# EUROPEAN PATENT APPLICATION

(11) **EP 3 949 886 A1**
(43) Date of publication of application: **09.02.2022**
(21) Application number: 20728092.6
(22) Date of filing: 03.04.2020
(51) Int. Cl.: A61B 18/20, A61N 5/06

(54) **NON-SURGICAL SYSTEM FOR THE RETRACTION OF A PATIENT'S EYELID TISSUE, THAT CAN BE COUPLED TO ANY LASER GENERATOR**

(30) Priority: 05.04.2019 ES 201930313
(71) Applicant: Intermedic Arfran, S.A., 08290 Cerdanyola del Vallès (ES)
(72) Inventor: QUEZEL-GUERRAZ, Jérome, 08290 CERDANYOLA DEL VALLÈS (ES); ARCUSA VILLACAMPA, Francisco Javier, 08290 CERDANYOLA DEL VALLÈS (ES)
(74) Representative: Ponti & Partners, S.L.P
(86) International application number: PCT/ES2020/070223
(87) International publication number: WO 2020/201604

(57) **Abstract**

The invention relates to a non-surgical system for the retraction of the eyelid tissue of a patient meant to tighten the tissue without making an ablation, that is designed to be coupled to any laser generator equipment present in the market and wherein the laser generator supplies a laser beam in continuous mode to a handpiece.

The invention also relates to said handpiece.

The system is characterised by comprising a handpiece that can be coupled to a laser generator operating in a power range of 0.1 to 10W and with treatment times of 0.1 to 5s, and having one or more lenses that focus or collimate the laser beam to a small spot with a diameter of 0.5 to 5mm. The system also comprises a timed pedal assembly with an electronic control unit associated with a timed pedal meant to compensate the delay time (tᵣ) of the mechanical shutter and add it to the programmed emission time on the skin (tₚ), thereby compensating said delay time (tᵣ) and allowing to obtain compensated laser beam pulses (t_{c}), as well as incorporating a timed module to allow obtaining the required laser beam pulse times of 0.1 to 5s.

## Description

The invention relates to a non-surgical system for retraction of the eyelid tissue of a patient, specifically designed to be coupled to any laser generator existing in the market at this time (or in the future), the aim of which is allowing a surgeon to perform a non-surgical retraction of the eyelid skin to tighten the eyelid tissue without making an ablation (i.e. a wound) when there is a low or medium excess of skin in the eyelid tissue.

The invention also relates to a non-surgical apparatus for retraction of the eyelid tissue of a patient, which the system comprises, and a laser generator provided with a mechanical shutter and wherein the laser generator supplies a continuous-mode laser beam that crosses the handpiece.

It also relates to a handpiece suitable for the non-surgical system for retraction of the eyelid tissue of a patient that is the main objective of the present invention.

### Background of the Invention

There is currently a high demand for blepharoplasty in oculoplastic ophthalmology, plastic surgery, maxillofacial surgery, dermatology or cosmetic medicine. Blepharoplasty can be performed on the upper or lower eyelids.

Throughout this specification, the general term "eyelid" or "eyelids" refers to both the upper and lower eyelid of a patient.

Blepharoplasty of the upper eyelid consists in eyelid surgery that seeks to rejuvenate the eyelid by remodelling periocular tissue, removing excess skin from the eyelids (particularly characteristic in middle-aged persons) and suturing so that the eyes recover a youthful appearance. It is mainly performed for aesthetic reasons, but in some cases, it is performed solely for visual reasons (functional blepharoplasty) as the falling skin from the upper eyelids can reduce the visual field.

Blepharoplasty of the lower eyelid consists in eyelid surgery meant to remove fatty tissue al change the collagen under the eyes, thereby eliminating bags under the eyes.

In general, blepharoplasty is performed using local anaesthesia and, in many cases, intravenous sedation to increase patient comfort. General anaesthesia is not required by the surgical procedure itself, but it is sometimes used when other more invasive interventions are performed simultaneously. The duration of the intervention is highly variable, between 30 minutes and 2 hours in general. Recovery after surgery is characterised by the appearance of oedema and ecchymosis (bruising) that improve with the application of cold in the first week. Full recovery can take from 3 to 6 weeks, and scars improve for 6 to 8 months.

One of the main drawbacks of this surgery known as blepharoplasty is that an incision and suture points are made in the treated area, which also produces a lesion in the treated skin that necessarily implies an extended postoperative period for the patient.

Patients increasingly demand dermoaesthetic procedures that are as non-invasive as possible. Consequently, when there is not a great excess of skin in the patient's eyelid blepharoplasty surgery can be satisfactorily replaced with a non-surgical retraction of the skin.

Lasers have been used for years in cosmetic surgery to treat other skin conditions and to eliminate fatty deposits in patients in areas other than the eyelids. For example, laser energy can be used for neck lifts or for reducing naso-labial folds. This type of laser treatments use a handpiece connected to a laser generator equipment. The laser can operate in continuous or pulse mode.

One of the problems addressed is that the handpiece of a laser equipment used to treat other parts of the body must be designed specifically for each laser equipment, significantly increasing the cost of the system as a whole. Therefore, the aim is to design a handpiece that is universal for all types of laser generator equipments existing in the market.

Consequently, it is necessary to provide an alternative to the prior art that covers the drawbacks found in same, by a non-surgical and minimally invasive system for retracting tissue of the upper or lower eyelid (that is, to restore a youthful appearance to the patient's eyelid) and wherein the system comprises a universal handpiece that can be coupled to any laser generator currently existing in the market.

### Description of the Invention

The main objective of the present invention, as defined in claim 1, is providing a non-surgical system for the retraction of eyelid tissue of a patient (in the upper and lower eyelids) that can be coupled to a laser generator of any type, preferably a CO₂ or diode laser generator, to achieve the retraction of the eyelid tissue (without ablation) using a laser beam generated in the corresponding laser generator that is aimed and focussed with the handpiece of the present invention at a specific treatment area in the patient's eyelid, thereby solving the aforementioned drawbacks and presenting the advantages described below.

To this end, the non-surgical system for retraction of eyelid tissue in a patient adapted to be coupled to any laser generator provided with a mechanical shutter, where the laser generator supplies a continuous mode laser that crosses internally a handpiece, characterised in that the non-surgical system of the invention, comprises:
- a handpiece provided with an outer hollow body having a distal part and a proximal part, wherein the distal part comprises an opening that can be coupled to a laser generator operating in a low power range from 0.1 to 10W and with high treatment times from 0.1 to 5 seconds, wherein the proximal part comprises an opening for outlet of the focussed or collimated laser beam towards the skin (15) of the eyelid of the patient, and where the handpiece further comprises at least the following elements:
   - one or more lenses arranged at fixed respective distances (dᵢ) from the proximal end (8) inside the outer body of the handpiece in a position perpendicular to the longitudinal axis of the outer body, which is/are configured to focus or collimate the laser beam to a small spot with a diameter from 0.5 to 5mm that is applied to the tissue of the eyelid with sufficient power density to heat the intra- and extra-cellular water in the eyelid tissue without making it boil; and
- a timed pedal assembly, comprising:
   - A timed pedal that replaces the original pedal of the laser generator,
      An electronic control unit associated with the timed pedal and configured to compensate for the delay time (tᵣ) characteristic of the mechanical shutter of the laser generator, and add it to the programmed emission time on the skin (tₚ) to compensate for this delay time (tᵣ) and allow obtaining compensated laser beam pulses (t_{c}) that exit the electronic control unit, and
   - a timer module connected to the electronic control unit and the general laser equipment, wherein the timer module is configured to allow obtaining laser beam pulse times from 0.1 to 5 seconds, which are high enough to perform this technique, and which the software of conventional laser generator do not normally provide.

In Spanish the term "circle" is used for the English term "spot".

Preferably, the system proposed by this invention is configured to be used coupled to any laser generator, such as a CO₂ laser generator, although it can also be coupled satisfactorily to other types of lasers, such as fibre lasers, diode lasers and the like. Specifically, CO₂ laser generators are gas lasers that use a gaseous mix of carbon dioxide that is electrically stimulated. They generate infra-red (non-visible) light with a wavelength of 10.6 micrometres. CO₂ lasers have a relatively high efficiency and very good beam quality, and consequently can be used satisfactorily in this treatment of the eyelid tissue.

A second aim of the present invention is providing an adapted handpiece for the non-surgical system for retraction of eyelid tissue of a patient that is the main aim of the present invention, wherein this handpiece is characterised in that it comprises, at least, the following elements:
- An outer body with a distal part and a proximal part, wherein the distal part comprises an opening that can be coupled to any laser generator operating in continuous mode in a low power range from 0.1 to 10W and with long treatment times from 0.1 to 5 seconds; and
- One or more focusing lenses arranged at fixed at a specific distance inside the outer body of the handpiece in a position perpendicular to the longitudinal axis of the outer body, which is/are configured to focus or collimate the laser beam to a small spot with a diameter from 0.5 to 5mm that is applied to the tissue of the eyelid with sufficient power density to heat the intra- and extra-cellular water in the eyelid tissue without making it boil.

A person's skin comprises approximately 70% water. Skin tissue responds to heat depending on the temperature reached by the tissue. The inventors of the present invention have found that the temperature range of 50 to 90°C (depending on the power density applied, that is, the combination of the power, spot and pulse duration) is best for stretching the skin by denaturalisation of the collagen. Under 50°C poor results are obtained, while over 90°C there may be skin necrosis or irreversible aesthetically undesirable results.

The inventors of the present invention have also found that if circles are made on the outer part of the eyelid skin using a laser with a wavelength that is well absorbed by water (that is, in the range 400 to 12,000nm) the intra- and extra-cellular water is heated, raising the tissue temperature to a temperature from 50 to 90°C, which will lead to the retraction of the eyelid skin. This retraction will tighten the skin and produce an effect similar to surgically cutting the excess skin and suturing it. However, as there are no incisions or suture points, and no lesion is caused, the postoperative period will be much shorter and more comfortable for the patient.

The temperature range for the eyelid tissue at the time of treatment with the system of the invention produces optimum eyelid retraction results, as it heats the intra- and extra-cellular water in the tissue without reaching boiling point, which would ablate the tissue and cause irreversible burns, leading to a wound that would require a longer postoperative period and cause undesired adverse effects.

A high power density requires obtaining a small treatment spot, with a diameter from 0.5 to 5mm.

According to a preferred embodiment of this invention the wavelength used in this treatment is between 1,400 and 11,000nm, as this range is well absorbed by water.

It is very important that the treatment can be repeated, so that it is essential that the size of the spot on the skin remains constant throughout the treatment so that the thermal effect (result) is also constant. For this purpose, the laser beam is focussed into a small spot using one or more lenses.

To ensure that the spot size remains constant throughout treatment, the handpiece of the system of the invention must remain in a perpendicular position and at a fixed distance from the skin of the patient to be treated.

To ensure that the spot size remains constant throughout the treatment, in a first preferred embodiment of this invention the handpiece incorporates in the proximal part thereof a part known as a spacer or mechanical reference, which rests on the skin of the patient to keep a constant working distance and therefore a constant spot surface area. This is the solution employed when a single focussing lens is used, such as a plano-convex lens. The spacer or mechanical reference element is placed at the proximal part of the outer body of the handpiece, where the spacer is meant to rest its free end on the skin of the patient to maintain a constant working distance and therefore a constant spot surface area, thereby ensuring that the size of the spot remains constant throughout the treatment. In this first embodiment the focusing lens is placed at a fixed focal length f₁ inside the outer body of the handpiece and fully perpendicular (at 90°) to the longitudinal axis of the outer body of the handpiece. The focal length of a lens is understood to be the distance between the optical centre of the lens and the focus (or focal point). In this first case the focusing lens, which is a convergent lens, focuses the laser beam from the laser generator in a specific point and then the beam is kept parallel along the spacer in a small circle with a diameter between 0.1 and 5mm, which is applied on the patient's skin.

Another option provided by this invention to keep the size of the spot applied on the patient's skin constant throughout the treatment (and therefore obtain a constant clinical result) without being affected by the separation of the handpiece from the tissue is for the interior of the handpiece to have a group of lenses spaced from each other to collimate the laser beam (which is different from focussing it as in the first embodiment. The term collimate means to reduce the size of the laser beam to the desired size of 0.5 to 5 mm while keeping the beam parallel in its exit path. In this second preferred embodiment of the invention, the handpiece incorporates two or more focussing lenses (preferably 2 or 3 lenses) arranged in fixed positions at different specific distances (dᵢ) inside the outer body of the handpiece and in a position perpendicular to the longitudinal axis of the outer body. In this second case a first focussing lens is a convergent lens used to focus the parallel laser beam arriving from the laser generator into a small spot with a diameter from 0.5 to 5mm, and one or more subsequent lenses (such as divergent lenses) that are arranged towards the proximal end meant to collimate the laser beam leaving the handpiece. In this case the spacer is no longer needed as the presence of the at least one divergent second lens collimates the laser beam, so that the proximal end does not need to touch the patient's skin.

Clinical experience has shown that to achieve retraction of the eyelid tissue without ablation it is necessary to operate in a relatively low power range (from 0.1 to 10W), with relatively small spots (0.1 to 5mm) and relatively long treatment times (0.1s to 5s).

Another aim of this invention, defined in claim 6, is that the handpiece for the non-surgical retraction of tissue can be used universally, that is, can be used with any laser generator existing in the market, preferably a laser generator that can operate in continuous mode. Most lasers can emit in continuous and pulsed modes.

One of the problems encountered by the owner of the present invention when attempting to couple the handpiece to an existing laser generator is that conventional laser generators are not ready to generate such long pulses, since they normally do not exceed 100ms.

Advantageously, this invention allows coupling the handpiece of the present invention and performing the non-invasive technique with any existing laser without having to modify its internal structure in any way.

For this purpose, the system of the invention incorporates a timer module that allows obtaining the long pulses required by this technique.

Most laser generator can emit in both continuous and pulsed mode. However, as mentioned before, in pulsed mode very few can provide pulses longer than 0.1ms. Consequently, to obtain the long pulses that are necessary the laser generator used in the non-invasive technique of the invention must be programmed in continuous operating mode.

The continuous operating mode, which is available in almost all laser generators in the market, is an operating mode that allows the pedal to directly determine the emission of the laser beam. This emission can be as short as the time for which the pedal is pressed, or infinitely long.

All laser equipments are provided as the actuation mechanism with an electro-mechanical pedal connected to the laser equipment to initiate treatment, and which the surgeon must press to start any treatment. To use this non-invasive technique proposed by the invention it is only necessary to disconnect the original pedal from the laser equipment and connect instead the timed pedal that can be supplied with the handpiece of the invention, and which is meant to time the laser pulses.

Another drawback solved by the present invention is that most laser generators are provided with a mechanical shutter for safety reasons, which has a non-instantaneous reaction time. That is, a few tenths of second elapse between the time that the pedal is pressed by the surgeon and the time that the mechanical shutter opens and the laser emission exits. Each mechanical shutter of each laser equipment has a specific delay time that can vary from one equipment to another.

Advantageously, the timed pedal of the present invention compensates said delay of the mechanical shutter by the electronic control unit of the timed pedal, preventing the reduction of the useful emission time on the skin required by the surgeon to treat a specific area of the eyelid.

This electronic control unit of the timed pedal compensates the delay time of the mechanical shutter for the specific laser equipment and adds this delay time to the time needed to treat the skin, thereby advantageously compensating the delay of the mechanical shutter which would make the emission time on the skin programmed for a patient to be different from that needed, which could have negative consequences if the desired eyelid retraction results are not attained.

Additional details and features will be explained in the description below with reference to the drawings appended to the present specification, which show, by way of a non-limiting example, a graphical representation of the invention.

### Brief description of the drawings

In order to better understand the description made, a set of drawings has been provided which, schematically and solely by way of non-limiting example, represent practical cases of various embodiments.
Figure 1 is a cross sectional view of the first embodiment of the handpiece (1) of the invention having a single lens (3) showing the various component parts thereof.
Figure 2 is a cross sectional view of the second embodiment of the handpiece (1) of the invention having two lenses (3, 4) showing the various component parts thereof.
Figure 3 is a side elevation view of a possible embodiment of the exterior of the handpiece (1) object of the invention.
Figure 4 is the same exterior view of the handpiece (1) as figure 3, in this case shown in a perspective view.
Figure 5 is a schematic view of the timed pedal assembly (11, 12, 13) connected to the general laser equipment (14).
Figure 6 is a representation of a possible timeline for the operation of the system of the invention.

### Description of Preferred Embodiments

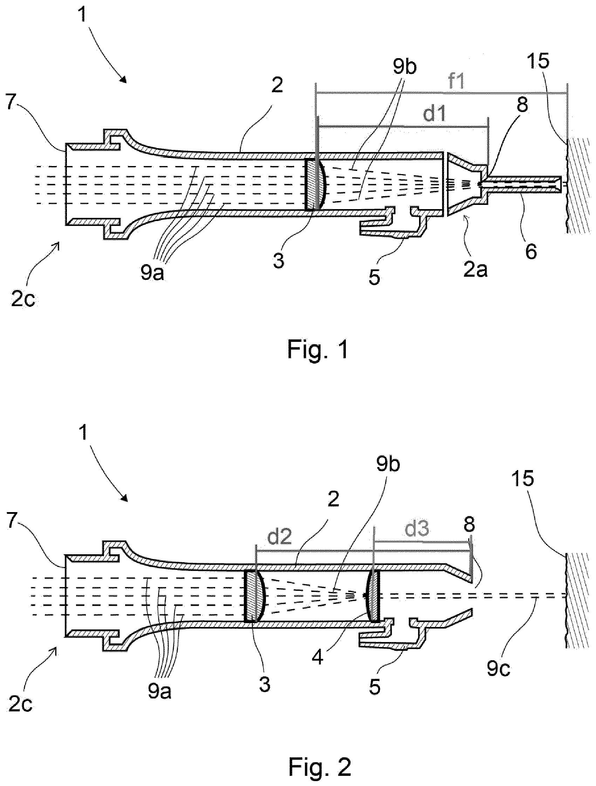

Two different embodiments are described below for the handpiece (1) of the present invention, with specific reference to Figures 1 to 4.

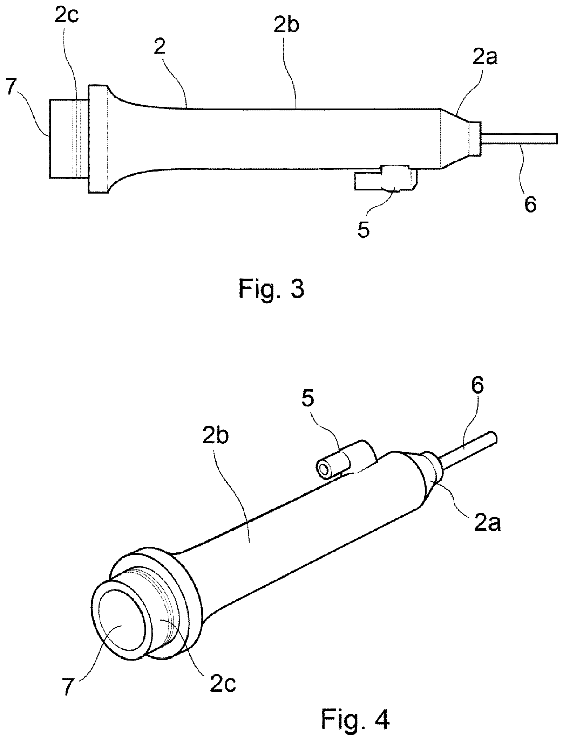

As can be seen in Figures 1 to 4, this invention relates to a system that incorporates a handpiece (1) which presents an outer body that is hollow on the inside (2, 2a, 2b, 2c) having a distal part (2c) and a proximal part (2a). This handpiece (1) can have a length, by way of example, between 4 and 20 cm. The distal part (2c) presents attachment means (such as threading, see figures 3 and 4) for coupling the opening (7) to the connector of a conventional laser generator (11, 12, 13, 14) which for this technique operates in a low power range from 0.1 to 10Wwith high pulse times between 0.1 and 5 seconds. The proximal part (2a) presents a narrowing that ends at a proximal opening (8) through which the focussed or collimated laser beam exits, as applicable, in the case of one or more lenses respectively. The handpiece (1) also incorporates an optional air inlet (5) meant to cool the inner part of the outer body (2).

In the first embodiment of the invention shown in figure 1, the handpiece (1) incorporates inside the outer body (2) a single plano-convex focusing lens (3) fixed in a position perpendicular to the longitudinal axis of the outer body (2) at a specified focal length (f₁). In this first case the focal length (f₁) of the lens (3) is preferably from 10mm to 200mm. In this case the proximal end incorporates a spacer (6) that can be integrated with the proximal part (2a) or be a separating element. The spacer (6) is an elongate piece with a suitable specific length which rests on the skin (15) of the patient and ensures that the working distance is always constant from the skin (15) of the patient, to ensure that the spot size remains constant throughout the treatment. As indicated above, the spacer (6) can be integrated in the outer body (2) or can be removable to allow cleaning. Before the laser beam (9a) reaches the focussing lens (3) presents a greater diameter on the skin (15) of the eyelid of the patient, and after crossing the convergent focussing lens (3) the outgoing laser beam (9b) is focussed (reduced) to the desired size (0.5 to 5 mm) on the skin (15) of the patient's eyelid.

In the second embodiment of the invention shown in figure 2, the handpiece (1) incorporates inside the outer body (2) two focussing lenses (3, 4) arranged perpendicular to the longitudinal axis of the outer body (2) and at specific respective distances (d2, d3) to the proximal opening (8), different from each other with respect to the proximal end (8). In this second case the beam exits the second focussing lens in collimated form, so that there is no focal length as the spot remains unchanged from a separation of 1cm to about 50cm.

The second lens (4), which is the one closest to the proximal area, collimates the inbound laser beam (9b) (that is, shrinks the laser beam to the desired size of 0.5 to 5 mm and keeps it parallel in its outbound path (9c)). Therefore, if there are more than two lenses (3, 4) the spacer is not necessary and is instead optional. In figure 2 no spacer is shown.

In both cases discussed above (figures 1 and 2) the lens(es) (3, 4) are configured and arranged inside the outer body (2) of the handpiece (1) set at the necessary distances with respect to the proximal end to focus or collimate (according to the case of figure 1 or figure 2) the laser beam into a small spot with a diameter between 0.5 and 5mm, on the eyelid tissue (15).

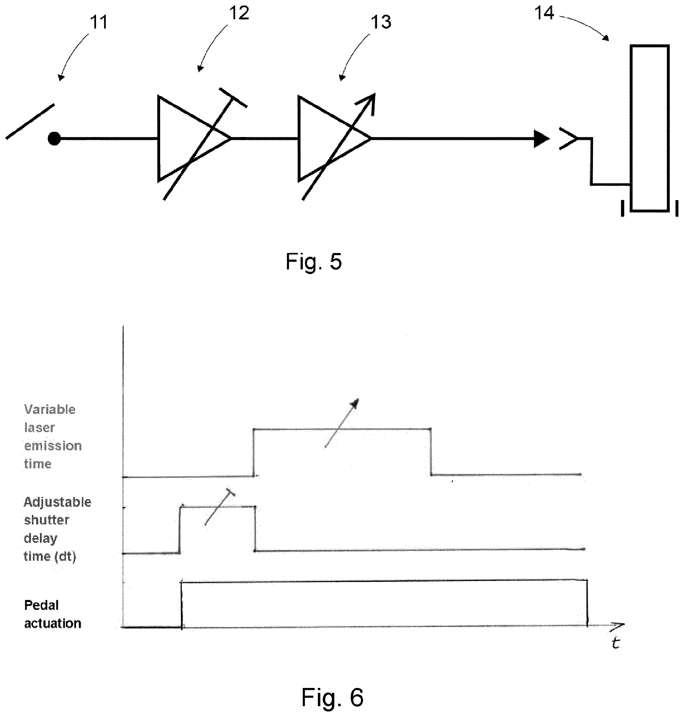

Figure 5 shows schematically the timed pedal assembly (11, 12, 13) of the invention, which is connected to the general laser equipment (14).

Figure 6 shows a possible timeline of the action of the system of the invention, a possible example of the pedal control unit based on a device that adjusts the delay time (tᵣ) of the mechanical shutter of the laser generator in question (performed only the first time), and the control of the emission time adjustable by the surgeon. The controls can be analogue or digital.

Although reference has been made to a specific embodiment of the invention, it is clear to a person skilled in the art that the system that can be coupled to any laser generator, equipment and handpiece described is susceptible to numerous variations and modifications, and that all the details mentioned can be replaced by other technically equivalent ones, without departing from the scope of protection defined by the attached claims.

## Claims

1. A non-surgical system for the retraction of the eyelid tissue of a patient, adapted to be coupled to any laser generator provided with a mechanical shutter, and where the laser generator supplies a laser beam in continuous mode that crosses a handpiece, **characterised in that** the non-surgical system comprises:
- a hollow handpiece (1) provided with an outer body (2, 2a, 2b, 2c) having a distal part and a proximal part, wherein the distal part comprises an opening (7) that can be coupled to a laser generator operating in a low power range between 0.1 and 10W and with high pulse times between 0.1 and 5 seconds, wherein the proximal part comprises an opening (8) for the exit of the laser beam, focussed or collimated, toward the skin (15) of the patient's eyelid, and where the handpiece (1) also comprises at least the following elements: one or more lenses (3, 4) arranged fixedly at respective distances (dᵢ) from the proximal end inside the outer body (2) of the handpiece (1) and in a position perpendicular to the longitudinal axis of the outer body (2), which are configured to focus or collimate the laser beam to a small circle with a diameter between 0.5 and 5mm that is applied to the tissue of the eyelid (15) with sufficient power density to heat the intra- and extra-cellular water of the eyelid without reaching the boiling point; and
- a timed pedal assembly, comprising:
- a timed pedal (11) that replaces the original pedal of the laser generator,
- an electronic control unit (12) associated with the timed pedal (11) and configured to compensate for the delay time (tᵣ) characteristic of the mechanical shutter of the laser generator, and add it to the programmed emission time on the skin (tₚ) to compensate for this delay time (tᵣ) and allow obtaining compensated laser beam pulses (t_{c}) that exit the electronic control unit, and
- a timer module (13) that is connected to the electronic control unit (12) and to the general laser equipment (14), wherein the timer module (13) is configured to obtain laser beam pulse times between 0.1 and 5 seconds.

2. The non-surgical system for the retraction of the eyelid tissue of a patient, according to any of the preceding claims, **characterised in that** the handpiece (1) comprises, in the proximal part thereof, a spacer or mechanical reference (6) which, when resting on the skin (15), allows maintaining a fixed working distance and consequently a constant spot surface area, thereby ensuring that the size of the spot remains constant throughout the treatment.

3. The non-surgical system for the retraction of the eyelid tissue of a patient, according to any of the preceding claims, **characterised in that** the interior of the handpiece (1) comprises one, two or more lenses (3, 4) separated from each other to collimate the beam, wherein the lenses (3, 4) are arranged fixed at respective different specific distances (dᵢ) with respect to the proximal end inside the outer body (2) of the handpiece (1) and in a position perpendicular to the longitudinal axis of the outer body (2).

4. A non-surgical system for the retraction of the eyelid tissue of a patient, comprising the system defined in any of the preceding claims and a laser generator provided with a mechanical shutter, where the laser generator supplies a laser beam in continuous mode that crosses the handpiece (1), **characterised in that** the laser generator is configured so as to work with a laser with a wavelength between 400 and 12,000nm.

5. The non-surgical system for the retraction of the eyelid tissue of a patient, according to claim 4, **characterised in that** the preferred wavelength range is 1,400 to 11,000nm.

6. Handpiece (1) suitable for the non-surgical system for the retraction of the eyelid tissue of a patient defined in any of claims 1 to 3, **characterised in that** it comprises, at least, the following elements:
- an outer body (2) having a distal part (2c) and a proximal part (2a), wherein the distal part (2c) comprises an opening (7) that can be coupled to a laser generator operating in continuous mode in a low power range from 0.1 to 10W and with high treatment times from 0.1 to 5 seconds, and wherein the proximal part (2a) comprises an opening (8) for outlet of the focussed or collimated laser beam towards the skin (15) of the eyelid of the patient; and
- one or more focusing lenses (3, 4) arranged at fixed respective distances (dᵢ) from the proximal end inside the outer body (2) of the handpiece (1) and in a position perpendicular to the longitudinal axis of the outer body (2), which is/are configured to focus or collimate the laser beam to a small spot with a diameter from 0.5 to 5mm to be applied to the tissue (15) of the eyelid with sufficient power density to heat the intra- and extra-cellular water in the eyelid tissue without making it boil.
